# EUROPEAN PATENT APPLICATION

(11) **EP 4 144 320 A1**
(43) Date of publication of application: **08.03.2023**
(21) Application number: 21797251.2
(22) Date of filing: 27.04.2021
(51) Int. Cl.: A61B 34/30

(54) **GUIDE RAIL DEVICE AND MEDICAL ROBOT**

(30) Priority: 29.04.2020 CN 202010354298
(71) Applicant: Suzhou Kangduo Robot Co., Ltd., Suzhou, Jiangsu 215153 (CN)
(72) Inventor: WANG, Jianguo, Suzhou, Jiangsu 215153 (CN); WANG, Xiaowei, Suzhou, Jiangsu 215153 (CN)
(74) Representative: Zaboliene, Reda
(86) International application number: PCT/CN2021/090204
(87) International publication number: WO 2021/218954

(57) **Abstract**

The invention provides a guide rail device and a medical robot, and relates to the technical field of medical instruments. The guide rail device comprises two curvilinear guide rails, two sliders and a connecting block, wherein the two curvilinear guide rails are arranged in spacing; the two sliders are slidably connected with the two curvilinear guide rails, respectively; the two sliders are rotationally connected with the top of the connecting block, respectively; and the bottom of the connecting block is adapted to connecting with the medical robot. The curvilinear guide rail is arranged at an altitude above a patient to be operated, so as to solve problems of the existing floor-standing medical robots represented by laparoscopic surgical robots, such as inflexible walking, inconvenient operation, easy blocking of the view and occupation for a lot of ground space.

## Description

### Technical Field

The invention relates to the technical field of medical instruments, in particular to a guide rail device and a medical robot.

### Background Art

Laparoscopic surgery is a newly developed minimally invasive method, and is an inevitable trend of development of surgical methods in the future. With the rapid development of industrial manufacturing technology, the integration of related disciplines lays a firm foundation for the development of novel technologies and methods; and combined with increasingly skillful operations of doctors, many open surgeries in the past are substituted with endovascular surgeries now, thereby greatly increasing surgical selection chances.

Laparoscopic surgical robots are important equipment for the laparoscopic surgery; the existing medical robots represented by the laparoscopic surgical robots generally have a floor-standing structure, which has disadvantages of inflexible walking, inconvenient operation, easy blocking of the view, occupation for a lot of ground space and the like.

### Summary of the Invention

The invention aims to solve problems that the existing medical robots represented by laparoscopic surgical robots generally have a floor-standing structure, and the structure has problems of inflexible walking, inconvenient operation, easy blocking of the view, occupation for a lot of ground space and the like.

To solve the problems, the invention provides a guide rail device, comprising:
two curvilinear guide rails, which are arranged in spacing;
two sliders, which are slidably connected with the two curvilinear guide rails, respectively; and
a connecting block, wherein the two sliders are rotationally connected with the top of the connecting block, respectively; and the bottom of the connecting block is adapted to connecting with a medical robot.

Further, each curvilinear guide rail comprises a first arc section guide rail and a second arc section guide rail connected to each other; and an arc opening direction of the first arc section guide rail is opposite to that of the second arc section guide rail.

Further, each curvilinear guide rail comprises a guide rail body and a supporting part; the supporting part comprises a supporting edge; the supporting edge is connected with the guide rail body, has an elongated structure, and extends to one side of a length direction of the guide rail body; one side of the length direction of the guide rail body refers to one contrary or opposite sides of two guide rail bodies;

Each slider comprises a sliding mechanism, which comprises a walking wheel; and the walking wheel is slidably connected to the supporting edge.

Further, each curvilinear guide rail comprises a guide rail body and a supporting part; the supporting part comprises two supporting edges; the two supporting edges are respectively connected with the guide rail body; one supporting edge has an elongated structure and extends to one side of the length direction of the guide rail body; the other supporting edge has the elongated structure and extends to the other side of the length direction of the guide rail body; one side of the length direction of the guide rail body refers to one contrary or opposite sides of the two guide rail bodies; the other side of the length direction of the guide rail body refers to one opposite or contrary sides of the two guide rail bodies;

Each slider comprises a sliding mechanism, which comprises two walking wheels; and each walking wheel is slidably connected to a corresponding supporting edge.

Further, each slider comprises a slider body and a rotating block; each slider body is rotationally connected with the connecting block, respectively; the top of each slider body is rotationally connected with the rotating block, respectively; and the rotating block is connected with one sliding mechanism.

Further, the walking wheels are walking wheels; rims of the walking wheels are provided with annular wheel grooves; and the supporting edges are arranged in the wheel grooves to prevent the walking wheels from coming off when moving along the supporting edges.

Further, the walking wheels are rotationally connected with the rotating blocks.

Further, each slider body comprises a middle body and two side bodies; the two side bodies are symmetrically distributed on both sides of the middle body; a bottom surface of the middle body is lower than bottom surfaces of the side bodies; the rotating blocks are rotationally connected to the side bodies through a first rotating shaft and a first bearing; and the middle body is rotationally connected to the connecting block through a second rotating shaft and a second bearing.

Further, each slider comprises a brake; and the brakes are arranged on the rotating blocks.

In addition, the invention further provides a medical robot, comprising the guide rail device.

Further, the medical robot comprises a robot body; and the robot body is connected with the sliders of the guide rail device.

Further, the robot body comprises a lifting rod, rotating arms, telescopic rods and surgical manipulators, which are sequentially arranged from top to bottom; and the top of the lifting rod is connected with the bottoms of the sliders.

Further, the curvilinear guide rails and the sliders of the guide rail device and the robot body are sequentially arranged from top to bottom.

The medical robot plays the same role in the utility model as the guide rail device, so the medical robot will not be explained.

Compared with the prior art, the guide rail device provided by the invention has but is not limited to the following technical effects:
the curvilinear guide rails are arranged, for example, above an operating table or a hospital bed, i.e., at an altitude above a patient to be operated, so the curvilinear guide rails in such a state will not block the view of patients and doctors, nor occupy the space on the ground; by adopting such a "curvilinear" design that the sliders slide along the curvilinear guide rails, the sliders are capable of at least sliding in a two-dimensional plane together with the robot body by just moving the sliders to an appropriate position on the two-dimensional plane, thereby facilitating operation; meanwhile, two curvilinear guide rails and two sliders are provided to reduce the load of each slider and each curvilinear guide rail, so that the structure is more stable; and in addition, in such a suspending arrangement case, the medical robot will not be hindered by obstacles (such as the hospital bed) and is able to walk flexibly, thereby solving problems that the existing medical robot represented by the laparoscopic surgical robot generally has a floor-standing structure, and the structure has disadvantages of inflexible walking, inconvenient operation, easy blocking of the view, occupation for a lot of ground space and the like.

### Brief Description of the Drawings

Figure 1 is a schematic structural diagram of a guide rail device according to embodiments of the invention;
Figure 2 is an enlarged view of A in Figure 1; and
Figure 3 is a schematic structural diagram of a slider according to embodiments of the invention; and
Figure 4 is a schematic sectional view of a slider according to embodiments of the invention.

Description of reference numerals:
1-curvilinear guide rail, 11-guide rail body, 12-supporting edge;
2-slider, 21-slider body, 211-middle body, 212-side body, 213-second rotating shaft, 214-second bearing, 215-second shaft sleeve, 22-rotating block, 221-first rotating shaft, 222-first bearing, 223-first shaft sleeve, 224-first hexagonal nut, 23-walking wheel, 231-wheel groove, 24-brake, 241-socket head cap screw;
3-robot body, 31-lifting rod, 32-arm rotating bracket, 33-rotating arm, 34-telescopic rod, 35-surgical manipulator; and
4-connecting block.

### Detailed Description of the Invention

To make objects, features and advantages of the invention plainer and more understandable, embodiments of the invention will be described in detail below with reference to the accompanying drawings.

In description of the invention, it should be understood that azimuths or positional relationships indicated by terms "upper", "lower", "front", "rear" and the like are based on azimuths or positional relationships shown in the accompanying drawings, are only used for facilitating the description of the invention and simplifying the description, instead of indicating or implying that the referred devices or elements must have specific directions and be constructed and operated in specific directions, and therefore cannot be understood as a limitation to the invention.

Moreover, a Z-axis in the accompanying drawings represents a vertical direction, i.e., an upper-and-lower position, and a positive direction of the Z-axis (i.e., an arrow direction of the Z-axis) represents the upper, while a negative direction of the Z-axis (i.e., a direction opposite to the positive direction of the Z-axis) represents the lower;

An X-axis in the accompanying drawings represents a longitudinal direction of a horizontal plane and is perpendicular to the Z-axis, and the positive direction of the X-axis (i.e., an arrow direction of the X-axis) represents a front side, while the negative direction of the X-axis (i.e., a direction opposite to the positive direction of the X-axis) represents a rear side;

A Y-axis in the accompanying drawings represents a transverse direction of the horizontal plane and is perpendicular to the Z-axis and the X-axis, and the positive direction of the Y-axis (i.e., an arrow direction of the Y-axis) represents a right side, and the negative direction of the Y-axis (i.e., a direction opposite to the positive direction of the Y-axis) represents a left side;

A plane formed by the X-axis and the Z-axis is a vertical plane.

Meanwhile, it should be noted that meanings of the Z-axis, Y-axis and X-axis are only used for facilitating the description of the invention and simplifying the description, instead of indicating or implying that the referred devices or elements must have specific directions and be constructed and operated in specific directions, and therefore cannot be understood as the limitation to the invention.

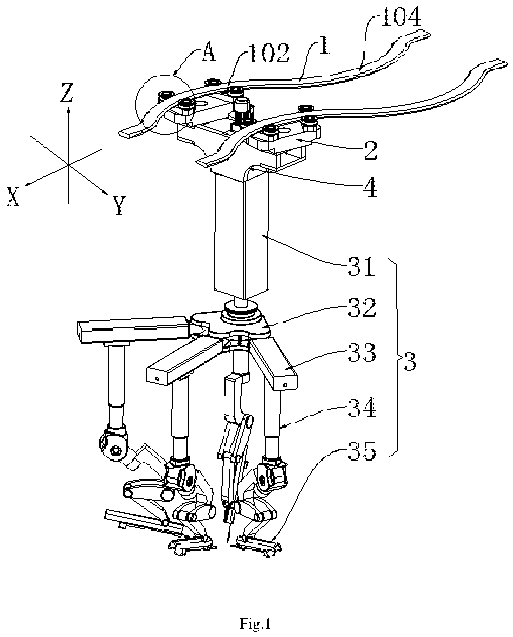

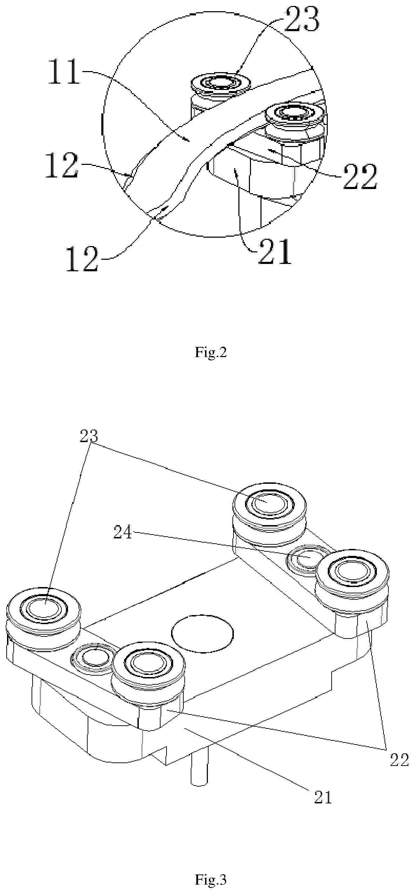

Referring to Figures 1 and 2, the present embodiment provides a guide rail device, used for a medical robot, wherein the guide rail device comprises:
curvilinear guide rails 1, wherein two curvilinear guide rails 1 are arranged in spacing;
a connecting block 4; and
sliders 2, wherein two sliders 2 are rotationally connected with two ends of the top of the connecting block 4 respectively, are slidably connected to the two curvilinear guide rails 1 respectively, and are adapted to moving along the curvilinear guide rails 1;

The medical robot is arranged at the bottom of the connecting block 4.

Here, the curvilinear guide rails 1 may be arranged at an altitude, which is defined relative to a patient to be operated; and the curvilinear guide rails 1 are arranged above an operation region of patient, may be arranged on a roof, and may also be arranged above a hospital bed or an operating table.

It should be noted that the guide rail device may be used not only for a laparoscopic surgical robot, but also for other medical robots. Here, taking the medical robot as an example, the laparoscopic surgical robot further comprises a robot body 3, wherein the robot body 3 is arranged at the bottom of the connecting block 4.

Here, the curvilinear guide rails 1 are arranged at an altitude above a patient to be operated, so the curvilinear guide rails 1 in such a state will not block the view of the patient and doctors, nor occupy the space on the ground; by adopting such a "curvilinear" design that the sliders 2 slide along the curvilinear guide rails 1, the sliders 2 are capable of sliding in a two-dimensional plane together with the robot body 3 by just moving the sliders 2 to an appropriate position on the two-dimensional plane, thereby facilitating operation; meanwhile, two curvilinear guide rails 1 and two sliders 2 are provided to reduce the load of each slider 2 and each curvilinear guide rail 1, so that the structure is more stable.

In addition, in such a suspending arrangement case, the robot body 3 will not be hindered by obstacles (such as the hospital bed) and is able to walk flexibly, thereby solving problems that the existing medical robot provided with a floor-standing structure and represented by the laparoscopic surgical robot has disadvantages of inflexible walking, inconvenient operation, easy blocking of the view, occupation for a lot of ground space and the like.

It should be noted that, here, not only the medical robot but other medical instruments in need of azimuthal change may be arranged at the bottoms of the sliders 2.

Meanwhile, it should be noted that the "arranged at" and "arranged in" include various connection modes such as fixed connection and detachable connection, and so do the "arranged at" and "arranged in" mentioned later in the present embodiment.

Referring to Figure 1, preferably, each curvilinear guide rail 1 comprises a first arc section guide rail 102 and a second arc section guide rail 104 connected to each other; and an arc opening direction of the first arc section guide rail 102 is opposite to that of the second arc section guide rail 104, i.e., the first arc section guide rail 102 and the second arc section guide rail 104 protrude toward different directions.

The first arc section guide rail 102 and the second arc section guide rail 104 are adopted to ensure that each curvilinear guide rail 1 is provided with at least two bends and the sliders 2 are capable of changing the direction at least twice in a two-dimensional plane, for example, being shown as an S type. Compared with the traditional linear guide rail, the curvilinear guide rail is more flexible and changeable in moving mode.

Referring to Figures 1 and 2, preferably, each curvilinear guide rail 1 comprises a guide rail body 11 and a supporting part; and the supporting part comprises a supporting edge 12.

When the supporting part comprises one supporting edge 12, the supporting edge 12 is connected with the guide rail body 11, has an elongated structure, and extends to one side of a length direction of the guide rail body 11, wherein one side of the length direction of the guide rail body 11 refers to one contrary or opposite sides of two guide rail bodies 11; each slider 2 comprises a sliding mechanism, which comprises a walking wheel 23; and the walking wheel 23 is slidably connected to the supporting edge 12.

The supporting edge 12 is arranged to provide a region for moving and supporting the sliders 2.

Here, the supporting edge 12 and the supporting body 11 may be integrally formed to reduce manufacturing cost and processing time.

When the supporting part comprises two supporting edges 12, the two supporting edges 12 are respectively connected with the guide rail body 11; one supporting edge 12 has an elongated structure and extends to one side of the length direction of the guide rail body 11; the other supporting edge 12 has the elongated structure and extends to the other side of the length direction of the guide rail body 11; one side of the length direction of the guide rail body 11 refers to one contrary or opposite sides of the two guide rail bodies 11; the other side of the length direction of the guide rail body 11 refers to one opposite or contrary sides of the two guide rail bodies 11; each slider 2 comprises a sliding mechanism, which comprises two walking wheels 23; and each walking wheel 23 is slidably connected to a corresponding supporting edge 12.

It should be understood that, compared with a structure that one supporting edge 12 is arranged, a structure that two supporting edges 12 are arranged and each supporting edge 12 is slidably connected with one walking wheel 23 may not only realize a basic requirement of moving along the curvilinear guide rails 1, but also ensure more stable movement.

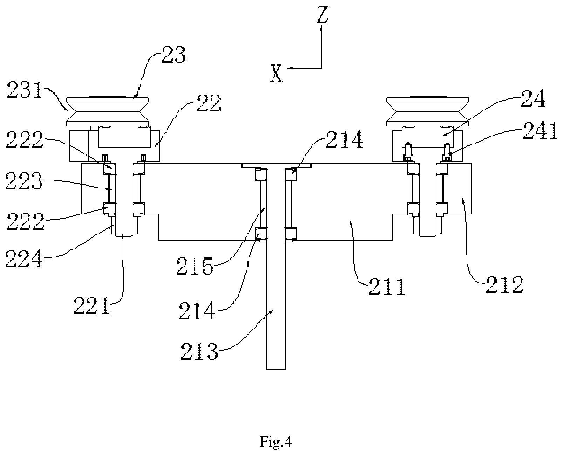

Referring to Figures 2 and 4, preferably rims of the walking wheels 23 are provided with annular wheel grooves 231; and the supporting edges 12 are arranged in the wheel grooves 231 to prevent the walking wheels 23 from coming off when moving along the supporting edges 12.

Referring to Figures 1-3, a specific structure of the sliders 2 is described here; preferably, each slider 2 further comprises slider bodies 21 and rotating blocks 22; each rotating block 22 is respectively connected with one sliding mechanism; each slider body 21 is rotationally connected with the connecting block 4 respectively; and the top of each slider body 21 is rotationally connected with two rotating blocks 22. Here, it should be noted that the walking wheels 23 are directly responsible for both load-bearing and guidance through cooperation between the wheel grooves 231 of the walking wheels 23 and the supporting edges 12, thereby, in a condition that the two curvilinear guide rails 1 correspond to two sliders 2, greatly reducing the load of the walking wheels 23 and ensuring the structural strength on the basis of ensuring stable walking and moving.

The rotary connection between the rotating blocks 22 and the slider bodies 21, and the rotary connection between the slider bodies 21 and the connecting block 4 are adopted to ensure that the rotating blocks 22 simultaneously rotate and change the direction when the slider bodies walk to radian regions of the curvilinear guide rails 1, so that the slider bodies can walk smoothly.

Referring to Figures 2 and 3, preferably, brakes 24 are arranged at the tops of the sliders 2; and the brakes 24 are adapted to stopping the sliders 2 from moving along the curvilinear guide rails 1.

Here, the brakes 24 may be electromagnetic power-off brakes. When the electromagnetic power-off brakes are powered off, the sliders 2 cannot continue to move on the curvilinear guide rails 1, thereby preventing the robot body 3 from unexpectedly moving and causing adverse consequences. The brakes 24 here are referred to the prior art, and are subject to realizing the braking of the sliders 2 on the curvilinear guide rails 1; for example, the brakes may also be friction brakes; and when the friction brakes are activated, the braking is realized by a friction force between braking parts of the friction brakes and the curvilinear guide rails 1, so as to keep a stop state.

Here, preferably, the brakes 24 may be arranged on the rotating blocks 22, for example, may be fixedly connected with the rotating blocks 22 through socket head cap screws 241; and the brakes 24 are located just below the guide rail bodies 11 after being arranged in position, thereby facilitating direct action on the guide rail bodies 11 to complete braking.

Referring to Figures 3 and 4, preferably, each slider body 21 comprises a middle body 211 and two side bodies 212; the two side bodies 212 are symmetrically distributed on both sides of the middle body 211; a bottom surface of the middle body 211 is lower than bottom surfaces of the side bodies 212; the rotating blocks 22 are rotationally connected to the side bodies 212 through a first rotating shaft 221 and a first bearing 222; and the middle body 211 is rotationally connected to the connecting block 4 through a second rotating shaft 213 and a second bearing 214.

Preferably, a side hole may be formed in each side body 212; the first rotating shaft 221 is inserted into the side hole to complete a relative rotation between the rotating block 22 and the side body 212; a first hexagonal nut 224 is screwed at a position where the bottom end of the first rotating shaft 221 extends out of the side hole; and the first hexagonal nut 224 is adopted to ensure that the rotating block 22 will not be separated from the side body 212.

Here, the first rotating shaft 221 may be integrally formed at the bottom of the rotating block 22; and the middle body 211 and the side bodies 212 may be integrally formed.

Here, the first bearing 222 may be arranged at both ends of the side hole; the first rotating shaft 221 is arranged in the first bearing 222 to complete the relative rotation with the side body 212; and one first bearing 222 may be arranged at each of upper and lower ends of the side hole, so that the rotation of the first rotating shaft 221 is more stable.

Here, the first bearing 222 may be a sliding bearing seat; a first shaft sleeve 223 is arranged in the middle of an inner wall of the side hole; the first shaft sleeve 223 is in interference fit with the sliding bearing seat; the first shaft sleeve 223 is in clearance fit with the first rotating shaft 221; and the first shaft sleeve 223 is provided to avoid the damage to the first rotating shaft 221.

Preferably, a middle hole may be formed in the middle body 211; the second rotating shaft 213 is inserted into the middle hole to complete the relative rotation between the middle body 211 and the connecting block 4; a second hexagonal nut is screwed at a position where the bottom end of the second rotating shaft 213 extends out of the bottom of the connecting block 4; the second hexagonal nut is adopted to ensure that the middle body 211 will not be separated from the connecting block 4, wherein the second rotating shaft 213 and the connecting block 4 may be fixedly connected, detachably connected or rotationally connected; and the connecting modes will not influence the relative rotation between the slider body 21 and the connecting block 4.

Here, the second bearing 214 may be arranged at both ends of the middle hole; the second rotating shaft 213 is arranged in the second bearing 214 to complete the relative rotation with the middle body 211; and one second bearing 214 may be arranged at each of upper and lower ends of the middle hole, so that the rotation of the second rotating shaft 213 is more stable.

Here, the second bearing 214 may be a sliding bearing seat; a second shaft sleeve 215 is arranged in the middle of an inner wall of the middle hole; the second shaft sleeve 215 is in interference fit with the sliding bearing seat; the second shaft sleeve 215 is in clearance fit with the second rotating shaft 213; and the first second sleeve 215 is provided to avoid the damage to the second rotating shaft 213.

Referring to Figure 3, preferably, the walking wheels 23 are rotationally connected with the rotating blocks 22.

The walking wheels 23 are rotationally connected with the rotating blocks 22, so that when the walking wheels 24 walk on the supporting edges 12, sliding friction is converted into rolling friction, thereby reducing resistance.

Referring to Figure 1, preferably, the curvilinear guide rails 1 may be of an S shape.

Here, the curvilinear guide rails 1 may be of an S shape; the S-shaped curvilinear guide rails 1 may not only provide movement of the sliders 2 on a horizontal plane, i.e., movement on an XY plane, but also have characteristics of attractive shape.

Referring to Figure 1, the present embodiment further provides a medical robot, which comprises the guide rail device; for example, the medical robot is a laparoscopic surgical robot; a robot body 3 of the laparoscopic surgical robot comprises a lifting rod 31, rotating arms 33, telescopic rods 34 and surgical manipulators 35, which are sequentially arranged from top to bottom; the top of the lifting rod 31 is connected with the bottoms of the sliders 2; the lifting rod 31 and the telescopic rods 34 are vertically arranged; and the rotating arms 33 are horizontally arranged.

Through a lifting function of the lifting rod 31, the rotating arms 33, the telescopic rods 34 and the surgical manipulators 35 are suspended highly when surgery is not needed; and the rotating arms 33, the telescopic rods 34 and the surgical manipulators 35 are moved down to a position slightly above the patient when surgery is needed.

Referring to Figure 1, preferably, a plurality of rotating arms 33 are provided; an arm rotating bracket 32 is arranged at the bottom of the lifting rod 31; and on a plane perpendicular to the lifting rod 31, one ends of the plurality of rotating arms 33 are rotationally connected into an accommodating groove of the arm rotating bracket 32.

The plurality of rotating arms 33 are provided, so that a plurality of surgical manipulators 35 may be provided; and the surgery may be completed more flexibly and simply under the action of the plurality of surgical manipulators 35 in different azimuths.

Referring to Figure 1, preferably, the telescopic rods 34 are adapted to moving along the length direction of the rotating arms 33.

The surgical manipulators 35 are more flexible through lifting of the telescopic rods 34 within a small range, rotation of the rotating arms 33 and movement of the telescopic rods 34 on the rotating arms 33.

It should be noted that a specific structure of the robot body 3 may be referred to the prior art and will not be repeated here.

Here, compared with the floor-standing structure, such a ceiling-mounted structure occupies a small space, has all wires at a high altitude, and has no cumbersome cables on the ground; through the lifting rod 31 on the Z-axis, the equipment may be lifted to the top end when the surgery is not needed, thereby having little influence on walking and view of personnel; the spatial movement of the surgical manipulators 35 is realized by the sliding of the sliders 2 on the curvilinear guide rails 1 and mutual movement of various joints, so as to accurately and conveniently position a wound position of a wounded person on the hospital bed; compared with a cantilever robot, the robot provided by the invention has a more stable and simpler structure, stable operating, accurate positioning and better dexterity, and can safely complete more delicate and complex operations.

Although the present disclosure is described above, a protection scope of the present disclosure is not limited to the above. Those skilled in the art may make various changes and modifications without departing from spirit and scope of the present disclosure; and all the changes and modifications will fall within the protection scope of the invention.

## Claims

1. A guide rail device, comprising:
two curvilinear guide rails (1), which are arranged in spacing;
two sliders (2), which are slidably connected with the two curvilinear guide rails, respectively; and
a connecting block (4), wherein the two sliders (2) are rotationally connected with the top of the connecting block (4), respectively; and the bottom of the connecting block (4) is adapted to connecting with a medical robot.

2. The guide rail device of claim 1, wherein each curvilinear guide rail (1) comprises a first arc section guide rail (102) and a second arc section guide rail (104) connected to each other; and an arc opening direction of the first arc section guide rail (102) is opposite to that of the second arc section guide rail (104).

3. The guide rail device of claim 1, wherein each curvilinear guide rail (1) comprises a guide rail body (11) and a supporting part; the supporting part comprises a supporting edge (12); the supporting edge (12) is connected with the guide rail body (11), has an elongated structure, and extends to one side of a length direction of the guide rail body (11); one side of the length direction of the guide rail body (11) refers to one contrary or opposite sides of two guide rail bodies (11);
each slider (2) comprises a sliding mechanism, which comprises a walking wheel (23); and the walking wheel (23) is slidably connected to the supporting edge (12).

4. The guide rail device of claim 1, wherein each curvilinear guide rail (1) comprises a guide rail body (11) and a supporting part; the supporting part comprises two supporting edges (12); the two supporting edges (12) are respectively connected with the guide rail body (11); one supporting edge (12) has an elongated structure and extends to one side of the length direction of the guide rail body (11); the other supporting edge (12) has the elongated structure and extends to the other side of the length direction of the guide rail body (11); one side of the length direction of the guide rail body (11) refers to one contrary or opposite sides of the two guide rail bodies (11); the other side of the length direction of the guide rail body (11) refers to one opposite or contrary sides of the two guide rail bodies (11);
each slider (2) comprises a sliding mechanism, which comprises two walking wheels (23); and each walking wheel (23) is slidably connected to a corresponding supporting edge (12).

5. The guide rail device of claim 3 or 4, wherein each slider (2) further comprises a slider body (21) and rotating blocks (22); each slider body (21) is rotationally connected with the connecting block (4), respectively; the top of each slider body (21) is rotationally connected with the rotating blocks (22), respectively; and the rotating blocks (22) are connected with one sliding mechanism.

6. The guide rail device of claim 3 or 4, wherein rims of the walking wheels (23) are provided with annular wheel grooves (231); and the supporting edges (12) are arranged in the wheel grooves (231) to prevent the walking wheels (23) from coming off when moving along the supporting edges (12).

7. The guide rail device of claim 5, wherein the walking wheels (23) are rotationally connected with the rotating blocks (22).

8. The guide rail device of claim 5, wherein each slider body (21) comprises a middle body (211) and two side bodies (212); the two side bodies (212) are symmetrically distributed on both sides of the middle body (211); a bottom surface of the middle body (211) is lower than bottom surfaces of the side bodies (212); the rotating blocks (22) are rotationally connected to the side bodies (212) through a first rotating shaft (221) and a first bearing (222); and the middle body (211) is rotationally connected to the connecting block (4) through a second rotating shaft (213) and a second bearing (214).

9. The guide rail device of claim 5, wherein each slider (2) further comprises a brake (24); and the brakes (24) are arranged on the rotating blocks (22).

10. A medical robot, comprising the guide rail device of any one of claims 1-9.

11. The medical robot of claim 10, comprising a robot body (3), wherein the robot body (3) is connected with the sliders (2) of the guide rail device.

12. The medical robot of claim 11, wherein the robot body (3) comprises a lifting rod (31), rotating arms (33), telescopic rods (34) and surgical manipulators (35) which are sequentially arranged from top to bottom; and the top of the lifting rod (31) is connected with the bottoms of the sliders (2).

13. The medical robot of claim 11, wherein the curvilinear guide rail (1) and the sliders (2) of the guide rail device and the robot body (3) are sequentially arranged from top to bottom.
